# EUROPEAN PATENT SPECIFICATION

(11) **EP 1 285 659 B1**
(45) Date of publication and mention of the grant of the patent: **27.06.2007**
(21) Application number: 01119436.2
(22) Date of filing: 13.08.2001
(51) Int. Cl.: A61K 31/7048, A61P 19/02

(54) **Use of clarithromycin compositions in the manufacture of a medicament for the treatment of arthritis deformans**
Verwendung von Clarithromycin zur Herstellung eines Medikaments für die Behandlung von Arthrosis deformans
Utilisation de la clarithromycine dans la fabrication d'un médicament pour le traitement de l'arthrite déformante

(43) Date of publication of application: 26.02.2003
(73) Proprietor: Fondazione Salvatore Maugeri - Clinica del Lavoro e della Riabilitazione, 27100 Pavia (IT)
(72) Inventor: Saviola, Gianantonio, 25010 Sirmione (Prov. of Brescia) (IT)
(74) Representative: Gervasi, Gemma

(56) References cited:
- WO-A-00/50620
- WO-A-01/49246
- WO-A-01/51059
- US-A- 5 985 890
- MATSUOKA N ET AL: "Inhibitory effect of clarithromycin on costimulatory molecule expression and cytokine production by synovial fibroblast-like cells." CLINICAL AND EXPERIMENTAL IMMUNOLOGY, vol. 104, no. 3, 1996, pages 501-508, XP002190408 ISSN: 0009-9104
- DARLING T N ET AL: "Treatment of Mycobacterium haemophilum infection with an antibiotic regimen including clarithromycin." BRITISH JOURNAL OF DERMATOLOGY, vol. 131, no. 3, 1994, pages 376-379, XP002190409 ISSN: 0007-0963
- SAVIOLA G. ET. AL.: "Clarithromycin in rheumatoid arthritis patients not responsive to disease-modifying antirheumatic drugs: An open, uncontrolled pilot study" CLINICAL AND EXPERIMENTAL RHEUMATOLOGY, vol. 20, 2002, pages 373-378,

## Description

### PRIOR ART

The arthritis deformans is a chronic syndrome usually characterized by symmetrical inflammation of the peripheral articulations which may give rise to the progressive destruction of the articular and periarticular structures.

The etiology of the arthritis deformans is not known, however an important factor from the pathogenic point of view consists of acatastasias of immunologic kind.

It is known that the treatment of the arthritis deformans is carried out by the administration of steroid and not steroid anti-inflammatory drugs, of gold compounds and cytotoxic drugs such as for example methotrexate, azathioprine and ciclosporin (The Merck Manual, Centennial Edition pp. 416-422).

However the serious side effects caused by the assumption of said drugs are known.

For example the steroid anti-inflammatory drugs may cause osteoporosis and vertebral compression fractures, hypokalemic alkalosis and edema, augmented susceptibility to infections and behavioristic diseases.

The not steroid anti-inflammatory drugs cause effects against the gastrointestinal apparatus and effects against the central nervous system such as for example vertigos.

The methotrexate exerts notable toxic effects against the gastrointestinal mucosa and against the medulla ossium and the liver, as well as causing the loss of hair.

For the treatment of the arthritis deformans even some antibiotics have been suggested but with poor results, as described for example by Pruzanski W. et al. in J. Rheumatol. 1992; 19: 1495-1496.

The prior art further discloses: tablet formulations comprising clarithromycin in the patent Application WO 01/49246; a process for preparing clarithromycin polymorphs in the patent Application WO 01/51059.

Moreover US 5,985,890 reports that rapamycin reduces swelling in an experimental model of developing arthritis Matsuoka et al. discloses that clarithromycin upregulates costimulatory molecule expression (Clin. Exp. Immunol., 1996, 104:501-508) while Darling et al. that clarithromycin is used for Mycobacterium haemophilum (British J. Dermatol. 1994 131: 376-379) treatment. WO 00/50620 claims that rheumatoid arthritis among other diseases, can be treated with an endokine-alpha antagonist.

On the other hand the use of a macrolide as clarithromycin for the treatment of gastric diseases associated to Helicobacter pylori is known, as described for example by Markham A. et al. in Drugs 1996; 51: 161-178.

### SUMMARY

Now we have surprisingly found that the administration of clarithromycin to patients suffering from arthritis deformans allows a substantial improvement of the disease with no relevant side effect.

Therefore the present invention refers to the use of clarithromycin for the preparation of pharmaceutical compositions suitable for the treatment of the arthritis deformans.

Optionally, the treatment with clarithromycin is associated to the treatment with a low dose cortisone-drug.

### DETAILED DESCRIPTION OF THE INVENTION

The present invention refers to the use of clarithromycin to prepare pharmaceutical compositions suitable for the treatment of the arthritis deformans having clarithromycin as active principle.

According to this use compositions can be formulated with excipients or diluents commonly used in the pharmaceutical technology and they are prepared for example in the form of pills.

A preferred formulation includes sodium crosscaramellosium, microcrystalline cellulose, silica gel, polyvinylpyrrolidone, stearic acid, magnesium stearate.

Preferably the pills have a hydroxypropylmethylcellulose covering film.

Each pill contains 500 mg of clarithromycin.

### CLINICAL EXPERIMENTATION

The clinical experimentation has been carried out on 18 patients of which 14 females and 4 males, having an average age equal to 62 years selected by the inclusion and exclusion criteria as hereinafter reported.

The patients submitted to the clinical experimentation suffered from arthritis deformans diagnosed according to the ARA International Criteria since at least two years, belonging to the 2^{th} and 3^{rd} functional classes according to Steinbrocker.

The disease was clinically active notwithstanding the use of drugs known as Disease-Modifying AntiRheumatic Drugs (DMARDS) able to modify the course of the illness such as for example methotrexate azathioprine, chloroquine, hydroxychloroquine and ciclosporin and notwithstanding the use of steroid and not steroid anti-inflammatory drugs.

The patients suffering from arthritis deformans of 4^{th} functional class according to Steinbrocker have been excluded from the experimentation that is the patients invalid with permanent injuries, the patients with associated serious systemic diseases, relevant psychiatric and neuropathic diseases, the drug addicts, the patients suffering from hypersensitivity to the macrolides and the patients under treatment with carbamazepine, ciclosporin and antihistamines.

The clarithromycin administration started after the suspension of the Disease-Modifying AntiRheumatic Drugs since one month.

In six patients who were assuming 3 steroid anti-inflammatory drug since at least two weeks, the administration of the same steroid drug with a 6 mg dose of prednisonic equivalent has been associated to the administration of clarithromycin. The administration of clarithromycin was 500 mg twice a day for 8-14 days and subsequently a customized dosage from 500 to 2,000 mg per day in one or two administrations has been applied.

The control of the patients has been periodically carried out on 0, 10, 30, 60, 90 and 180 days by medical examination and determination of the haematochemical and functional indexes.

In particular the following indexes have been controlled:
- HAQ (Health Assessment Questionnaire, 1986), internationally validated as reference functional index in the rheumatic disease;
- VAS (Visuanalogic Ache Scale according to Scott Huskisson);
- ESR (Erythro Sedimentation Rate).

The patients responding to the therapy were 14 while the remaining 4 were not responding.

A significant reduction of the symptomatology starting from the second week of treatment has been found.

No significant side effect ascribable to the drug in the whole experimentation period has been found.

The results relating to the above defined indexes are reported in the following Table 1.

**Table 1**

| Rheumatic Control Indexes | | | |
|---|---|---|---|
| Day | HAQ | VAS | ESR |
| 0 | 20.2±2.6 | 4.7±0.51 | 44.8±8.4 |
| 180 | 5.5±0.51 | 1.32±0.44 | 27±5.8 |
| | (a) | (b) | (c) |

| | | | |
|---|---|---|---|
| (a) p < 0.0001 (Mann-Whitney U test) (b) p < 0.05 (Mann-Whitney U test) (c) p < 0.05 (Mann-Whitney U test) | | | |

The above reported results show a high activity of clarithromycin in the treatment of the arthritis deformans thereby said drug may be successfully used for the preparation of compositions suitable for this treatment.

Said compositions include a pharmaceutically effective amount of clarithromycin in mixture with pharmaceutically acceptable excipients of normal use in pharmaceutical technology.

The therapeutic method provides for the administration by oral way of a 500 mg dose of clarithromycin per day as attack dose for 8 - 14 days and subsequently a customized maintenance dose from 500 to 2,000 mg per day in one or two administrations.

## Claims

1. Use of clarithromycin for the preparation of pharmaceutical compositions suitable for the treatment of the arthritis deformans.

2. Use as claimed in claim 1, **characterized in that** said compositions include a pharmaceutically effective amount of clarithromicin in mixture with pharmaceutically acceptable excipients.

## Patentansprüche

1. Verwendung von Clarithromycin für die Herstellung von pharmazeutischen Zusammensetzungen, die geeignet sind für die Behandlung von Arthritis deformans.

2. Verwendung wie in Anspruch 1 beansprucht, **dadurch gekennzeichnet, dass** die genannten Zusammensetzungen eine pharmazeutisch wirksame Menge an Clarithromycin in Mischung mit pharmazeutisch anerkannten Trägerstoffen beinhalten.

## Revendications

1. Utilisation de la clarithromycine pour la préparation de compositions pharmaceutiques qui conviennent pour le traitement de l'arthrite déformante.

2. Utilisation selon la revendication 1, **caractérisée en ce que** lesdites compositions comprennent une quantité pharmaceutiquement efficace de clarithromycine en mélange avec des excipients pharmaceutiquement acceptables.
